# EUROPEAN PATENT SPECIFICATION

(11) **EP 1 859 762 B1**
(45) Date of publication and mention of the grant of the patent: **14.07.2010**
(21) Application number: 07075410.6
(22) Date of filing: 26.04.2007
(51) Int. Cl.: A61F 5/03

(54) **Thoracic compression device**
Brustkompressionsvorrichtung
Dispositif de compression thoracique

(30) Priority: 27.04.2006 ES 200601080
(43) Date of publication of application: 28.11.2007
(73) Proprietor: Ruiz Escalera, Isabel, 08800 Vilanova i La Geltru (ES); Claramunt Artigas, Daniel, 08800 Vilanova i La Geltru (ES)
(72) Inventor: Ruiz Escalera, Isabel, 08800 Vilanova i La Geltru (ES)
(74) Representative: Esteban Perez-Serrano, Maria Isabel

(56) References cited:
- WO-A-97/06761
- US-A- 2 662 522
- US-A- 4 960 112
- US-A- 5 152 741
- US-A- 5 527 270

## Description

### OBJECT OF THE INVENTION

The present invention refers to a thoracic compression device. The device is designed for use in the field of medicine in cases where compression and holding of the thoracic zone is required, for example following breast surgery or in the case of rib injuries or any other situation requiring compression and hold.

### BACKGROUND TO THE INVENTION

It is well known that following surgical intervention of the breast or in the case of rib injuries it is necessary to compress and hold the damaged zone in order to facilitate recovery.

In breast operations this compression prevents haemorrhage, haematomas, seromas, etc. whereas in rib injuries it is used to provide support in the injured zone and to alleviate pain.

At present the function of compression is carried out by bandaging the patent with bands or gauze which may be self adhesive. This system has the disadvantage that when removing the bandage the patient is subjected to the pain produced by detaching the adhesive

The invention described here overcomes these disadvantages by means of a device which provides compression and hold which may be regulated both in terms of the area requiring the greatest compression and in the case in which greater pressure is required, for example in the hours immediately following surgical intervention. Another advantage of the present device is that it compresses a single haemothorax so that the undamaged rib would remain compressed, in this way, ensuring that the patient does not suffer any difficulty with breathing.

The invention described also has the advantage of the fact that by using a textile fabric which does not adhere to the patient's skin it avoids abrasions, skin rashes or blisters.

Nevertheless it is known in the state of the art the Thoracic compression devices as the one disclosed in US 5527270 A, US 5152741 A, US 4960112 A and US 2262522 A, which have in common that all of them have a main body which surrounding completely the torso of the patient, being covered both hemithorax, so both hemithorax are submitted to the same pressure, making difficult breathing.

Additionally it is also known in the state of the art an axillary dressing specially designed for breast cancer surgery and covers the axillary area, not covering a whole hemithorax which addtionally does not allow to regulate the horizontal compression exercised over the hemithorax.

The aforementioned drawbacks are overcome with the solution now proposed.

### DESCRIPTION OF THE INVENTION

The present invention refers to a thoracic compression device. The device is designed for use in the field of medicine in cases where compression and holding of the thoracic zone is required, for example following breast surgery or in the case of rib injuries or any other situation requiring compression and holding.

The device comprises the features specified in claim 1.

The means for regulating the compression exerted consists of the extension of the main body in bands which surround at least the damaged hemithorax until they link once more with the main body.

The means for holding the main body to the shoulder consists of a band with an adjustable height which starts from the main body and surrounds the shoulder.

### DESCRIPTION OF THE DRAWINGS

The present descriptive report is supplemented by a series of drawings illustrative of a preferred embodiment but not however restricting the invention in any way.

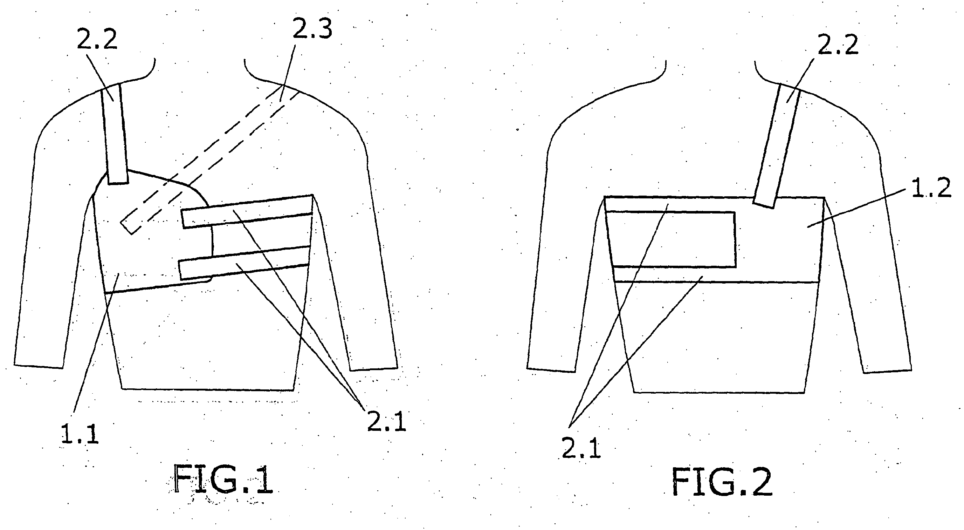

Figure 1 shows a diagram of the front part of the compression device.
Figure 2 shows a diagram of the back part of the compression device.
Figure 3 is a representation in extended section of the main body and the bands.

### PREFERRED EMBODIMENT OF THE INVENTION

The present invention refers to a thoracic compression device. The device described is applicable in the field of medicine, in those cases in which the thoracic area needs to be compressed and supported.

The device described is characterised by the features specified in claim 1.

The means for adjusting horizontal compression consists of the extension of the main body (1) into bands(2.1) which surround at least the undamaged hemithorax until they link again with the main body (1). The bands (2.1) start from the back area of the main body (1) and are joined thereto (1) in the front area.

The means for holding the main body (1) to the patient's shoulder also consist of a band (2.2) with an adjustable height which connects with the front part of the main body (1) with the back part passing over the shoulder.

The connection between the bands (2.1, 2.2) and the main body (1) is achieved through a physical adherence system or based on chemical products.

In the preferred embodiment the adhesive system consists of the combination of Velcro^{®} (hooks material portion and loops material) in the bands (2.1, 2.2) and a textile fabric of the main body (1) appropriate for adherence thereto. Thus, in a simple system based on the position in which the bands are stuck to the main body, the compression exerted by the device can be varied.

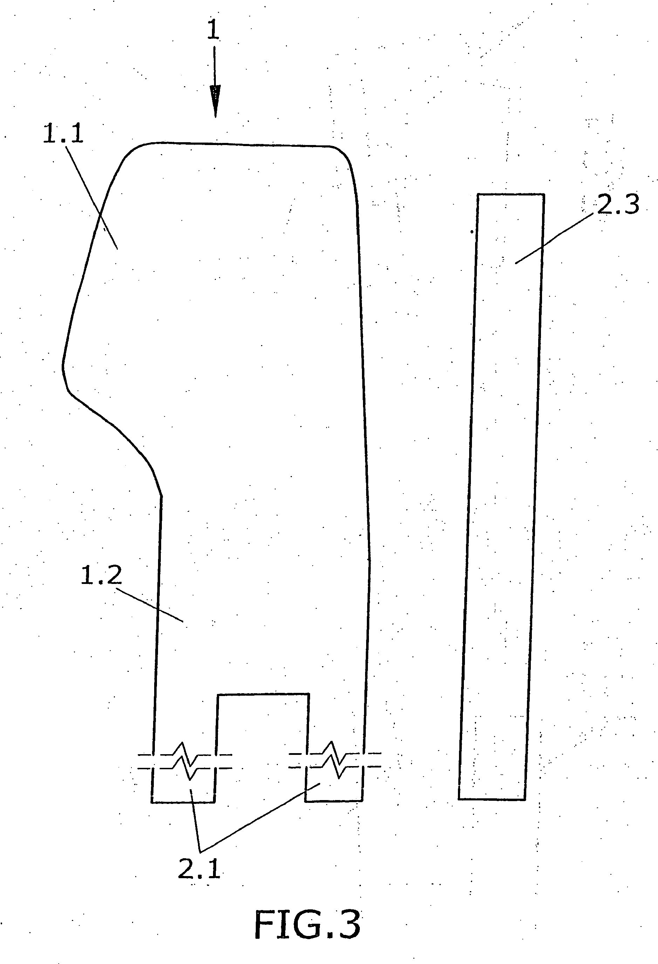

The main body (1) of the preferred embodiment is provided with a front zone (1.1) which is wider than the back zone (1.2) as may be seen in figure 3.

The device is also provided with bands of extra compression (2.3) with an adhesive surface at both ends which provide greater compression in those zones or at those times when this is required.

The main textile from which both the main body (1) and the bands (2.1, 2.2, 2.3) are made may be quilted for the patient's extra comfort

The essential nature of this invention is not altered in any way by variations in materials, form, shape and arrangement of the component elements which are described in a manner which is in no way restrictive but which is sufficient to for an expert to proceed to its reproduction.

## Claims

1. Thoracic compression device, comprising a main textile body (1) provided with means (2.1) for regulating the compression exerted and means (2.2) for holding it on at least one shoulder, **characterized in that** the main textile body is constructed to cover only one hemithorax, while the means for regulating the compression exerted consists of the extension of the main textile body (1) into bands(2.1) which are adapted to surround a healthy hemithorax until they link again with the main body (1).

2. Thoracic compression device according to claim 1 **characterised in that** the means for holding it to the shoulder consist of a band (2.2) with an adjustable length.

3. Thoracic compression device according to previous claims in which the connection between the bands, (2.1, 2.2) and the main body (1) is carried the out through an adhesive medium.

4. Thoracic compression device according to claim 3, **characterised in that** the band (2.2) which surrounds the shoulder is adhesive at both ends.

5. Thoracic compression device according to claim 3 **characterised in that** the adhesive medium consists of a combination of hooks material portion and loops material in the bands (2.1, 2.2) and a textile fabric of the main body (1) appropriate for adherence thereto.

6. Thoracic compression device according to claim 1, **characterised in that** the main body (1) is provided with a front area (1.1) with greater width than the back area (1.2).

7. Thoracic compression device according to claim 1, **characterised in that** it is provided with adhesive bands (2.3) which reinforce compression.

8. Thoracic compression device according to claim 1, **characterised in that** the fabric of the main body (1) may be quilted.

## Patentansprüche

1. Kompressionsvorrichtung des Brustkorbs, die einen textilen Hauptkörper (1) umfasst, der ausgestattet ist mit Mitteln (2.1) zur Regulierung der ausgeübten Kompression und Mitteln (2.2), um ihn an wenigstens einer Schulter zu halten, **dadurch gekennzeichnet, dass** der textile Hauptkörper dazu konstruiert ist, nur eine Brustkorbhälfte zu bedecken, während das Mittel zur Regulierung der ausgeübten Kompression aus einer Erweiterung des textilen Hauptkörpers (1) zu Gurten (2.1) besteht, welche angepasst sind, um die gesunde Brustkorbhälfte zu umrunden, bis sie wieder an den Hauptkörper (1) anschließen.

2. Kompressionsvorrichtung des Brustkorbs nach Anspruch 1, **dadurch gekennzeichnet, dass** die Mittel, um sie an der Schulter zu halten, aus einem Gurt (2.2) mit einer einstellbaren Länge bestehen.

3. Kompressionsvorrichtung des Brustkorbs nach den vorhergehenden Ansprüchen, in welcher die Verbindung zwischen den Gurten (2.1, 2.2) und dem Hauptkörper (1) durch ein haftendes Mittel ausgeführt wird.

4. Kompressionsvorrichtung des Brustkorbs nach Anspruch 3, **dadurch gekennzeichnet, dass** der Gurt (2.2), welcher die Schulter umrundet, an beiden Enden haftend ist.

5. Kompressionsvorrichtung des Brustkorbs nach Anspruch 3, **dadurch gekennzeichnet, dass** das haftende Mittel aus einer Kombination aus Hakenmaterialabschnitt und Schlingenmaterial in den Gurten (2.1, 2.2) und einem zur Haftung daran geeigneten textilen Gewebe des Hauptkörpers (1) besteht.

6. Kompressionsvorrichtung des Brustkorbs nach Anspruch 1, **dadurch gekennzeichnet, dass** der Hauptkörper (1) mit einem vorderen Bereich (1.1) mit größerer Breite als der hintere Bereich (1.2) ausgestattet ist.

7. Kompressionsvorrichtung des Brustkorbs nach Anspruch 1, **dadurch gekennzeichnet, dass** sie mit haftenden Gurten (2.3) ausgestattet ist, welche eine Kompression verstärken.

8. Kompressionsvorrichtung des Brustkorbs nach Anspruch 1, **dadurch gekennzeichnet, dass** das Gewebe des Hauptkörpers (1) gesteppt sein kann.

## Revendications

1. Dispositif de compression thoracique, comprenant un corps principal textile (1) muni de moyens (2.1) pour réguler la compression exercée et de moyens (2.2) pour lui soutenir sur au moins une épaule, **caractérisé en ce que** le corps principal textile est construit de manière à couvrir seulement un hémithorax, tandis que les moyens pour réguler la compression exercée se composent de la prolongation du corps principal textile (1) formant des bandes (2.1) qui sont adaptées pour entourer un hémithorax sain, jusqu'à ce qu'ils s'attachent de nouveau au corps principal (1).

2. Dispositif de compression thoracique, selon la revendication 1, **caractérisé en ce que** les moyens pour lui soutenir sur l'épaule se composent d'une bande (2.1) de longueur ajustable.

3. Dispositif de compression thoracique, selon les revendications préalables, dans lequel la connexion entre les bandes (2.1, 2.2) et le corps principal (1) s'effectue à l'aide d'un moyen adhésif.

4. Dispositif de compression thoracique, selon la revendication 3, **caractérisé en ce que** la bande (2.2) qui entoure l'épaule est adhésive dans les deux extrémités.

5. Dispositif de compression thoracique, selon la revendication 3, **caractérisé en ce que** le moyen adhésif se compose d'une combinaison de une portion de matériel à crochets et de matériel à boucles dans les bandes (2.1, 2.2) et un tissu textile du corps principal (1) approprié pour s'y adhérer.

6. Dispositif de compression thoracique, selon la revendication 1, **caractérisé en ce que** le corps principal (1) est muni d'une zone avant (1.1) de plus grande largeur que la zone arrière (1.2).

7. Dispositif de compression thoracique, selon la revendication 1, **caractérisé en ce qu'**il est muni de bandes adhésives (2.3) renforçant la compression.

8. Dispositif de compression thoracique, selon la revendication 1, **caractérisé en ce que** le tissu du corps principal (1) peut être molletonné.
